# EUROPEAN PATENT APPLICATION

(11) **EP 3 949 742 A1**
(43) Date of publication of application: **09.02.2022**
(21) Application number: 20778830.8
(22) Date of filing: 27.03.2020
(51) Int. Cl.: A23C 9/16, C12N 1/00, C12N 1/20

(54) **HEAT-RESISTANT BACTERIUM COMPOSITION**

(30) Priority: 28.03.2019 JP 2019063902
(71) Applicant: Morinaga Milk Industry Co., Ltd., Minato-ku Tokyo 108-8384 (JP)
(72) Inventor: OZAWA, Tomohito, Kanagawa 252-8583 (JP)
(74) Representative: Vossius & Partner Patentanwälte Rechtsanwälte mbB
(86) International application number: PCT/JP2020/014078
(87) International publication number: WO 2020/196844

(57) **Abstract**

A technique for improving heat resistance of a bacterium is provided. By mixing the bacterium with an oil or fat and an emulsifier, heat resistance of the bacterium can be improved.

## Description

### Technical Field

The present disclosure relates to a composition containing a bacterium having improved heat resistance, and a method of producing it.

### Background Art

Known examples of techniques for improving heat resistance of a bacterium include coating or capsulation of the bacterium utilizing a component such as a milk protein (Patent Document 1), a coagulant (Patent Document 2), a hydrophobic substance which is non-flowable at normal temperature (Patent Document 3), a stabilizer and a protecting agent (Patent Document 4), a protein-based edible gelling agent (Patent Document 5), a protein (Non-patent Document 1), or a low-melting-point oil or fat (LMF) (Non-patent Document 2).

Other examples of such techniques include coating or granulation of a bacterium utilizing a glycerin fatty acid ester (Patent Documents 6 and 7). However, Patent Documents 6 and 7 do not describe heat resistance of the bacterium.

It has not been known that, by mixing a bacterium with an oil or fat and an emulsifier, heat resistance of the bacterium can be improved.

### Prior Art Documents

### [Patent Documents]

Patent Document 1: Japanese Patent No. 3698455
Patent Document 2: JP-A-2004-344146
Patent Document 3: Japanese Patent No. 3102990
Patent Document 4: Japanese Patent No. 5841527
Patent Document 5: JP-A-10-191950
Patent Document 6: JP-A-2-200639
Patent Document 7: Japanese Patent No. 2596418

### [Non-patent Documents]

Non-patent Document 1: Journal of Food Science, 2017, 9, 2134-2141
Non-patent Document 2: Food Hydrocolloids, 2015, 51, 459-467

### Summary

### Problems to be Solved by the Invention

An object of the disclosure is to provide a technique for improving heat resistance of a bacterium.

### Means for Solving the Problems

The present inventors discovered that, by mixing a bacterium such as a bacterium belonging to the genus *Bifidobacterium* with an oil or fat and an emulsifier, heat resistance of the bacterium can be improved, thereby completing the invention.

More specifically, the invention can be exemplified as follows. One aspect of the invention is a method of producing a composition containing a bacterium, the method including the following Steps A and B:
(A) mixing the bacterium with an oil or fat; and
(B) mixing the bacterium with an emulsifier;
wherein the Step A is carried out at the melting point of the oil or fat or a higher temperature.

In a preferred aspect of the method, the Step A begins before or after beginning the Step B.

In a preferred aspect of the method, the Steps A and B begin at the same time.

In a preferred aspect of the method, the oil or fat has a melting point of 50°C or lower.

In a preferred aspect of the method, the oil or fat has a melting point of 35°C or higher.

In a preferred aspect of the method, the oil or fat is a fully hydrogenated palm kernel oil.

In a preferred aspect of the method, the Step A is carried out at a temperature of 50°C or lower.

In a preferred aspect of the method, the Step A is carried out at a temperature of 40°C or higher.

In a preferred aspect of the method, the emulsifier is a lecithin or polyglycerin fatty acid ester.

In a preferred aspect of the method, the Step B is carried out at a temperature of 50°C or lower.

In a preferred aspect of the method, the Step B is carried out at a temperature of 40°C or higher.

In a preferred aspect of the method, the amount of the oil or fat is 1.5 to 4.5 g per 50 g dry cell weight of the bacterium.

In a preferred aspect of the method, the emulsifier is a polyglycerin fatty acid ester, and the amount of the emulsifier is 8 to 17 g per 50 g dry cell weight of the bacterium.

In a preferred aspect of the method, the emulsifier is a lecithin, and the amount of the emulsifier is 1 to 20 g per 50 g dry cell weight of the bacterium.

In a preferred aspect of the method, the bacterium belongs to the genus *Bifidobacterium.*

In a preferred aspect of the method, the bacterium is *Bifidobacterium longum* or *Bifidobacterium breve.*

In a preferred aspect of the method, the bacterium is *Bifidobacterium longum* BB536 (NITE BP-02621) or *Bifidobacterium breve* M-16V (NITE BP-02622).

One aspect of the invention is a method of improving heat resistance of a bacterium, the method including the following Steps A and B:
(A) mixing the bacterium with an oil or fat; and
(B) mixing the bacterium with an emulsifier;
wherein the Step A is carried out at the melting point of the oil or fat or a higher temperature.

One aspect of the invention is a composition comprising a bacterium, an oil or fat, and an emulsifier.

In a preferred aspect of the composition, the bacterium is coated with the oil or fat and the emulsifier.

One aspect of the invention is a powdered milk comprising: a composition produced by the method described above; or the composition described above.

In a preferred aspect, the powdered milk is a powdered infant formula milk.

### Mode for Carrying Out the Invention

The invention is described below in detail. Unless otherwise specified, the following descriptions on the invention may be employed individually, or may be employed in an appropriate combination.

### <1> Method of Invention

The method of the disclosure is a method including the following Steps A and B:
(A) mixing the bacterium with an oil or fat; and
(B) mixing the bacterium with an emulsifier.

By the method of the disclosure, heat resistance of a bacterium can be improved. In other words, by the method of the disclosure, an effect wherein improved heat resistance of a bacterium can be obtained. The effect is also referred to as "heat resistance-improving effect". Thus, the method of the disclosure may be, for example, a method of improving heat resistance of a bacterium.

Furthermore, by the method of the disclosure, a composition including a bacterium may be obtained. The composition including a bacterium is also referred to as "bacterium composition". Thus, the method of the disclosure may also be, for example, a method of producing a bacterium composition. The bacterium contained in the bacterium composition may be a bacterium having improved heat resistance. Thus, the bacterium composition may be a composition including a bacterium having improved heat resistance. The composition including a bacterium having improved heat resistance is also referred to as "heatresistant bacterium composition". In one mode, the bacterium composition may be the later-mentioned composition of the disclosure.

The term "Improvement of heat resistance" means that the heat resistance of the bacterium after the operations of Steps A and B is higher than the heat resistance of the bacterium before the operations of Steps A and B. Examples of the improvement of heat resistance include improvement of the survival rate of the bacterium after heating of the bacterium. The heating temperature may be, for example, 70°C. The heating time may be, for example, 1 minute. Thus, more specifically, the heating may be carried out, for example, at 70°C for 1 minute. The survival rate after heating the bacterium at 70°C for 1 minute may be measured, for example, as the survival rate 1 minute after dispersion of the bacterium in hot water at 70°C. More specifically, the survival rate after heating the bacterium at 70°C for 1 minute may be measured, for example, under the conditions described in the Examples. The survival rate after heating the bacterium is calculated as the ratio of the viable cell count after heating to the viable cell count before the heating (more specifically, at the beginning of the heating). The viable cell count may be measured as, for example, the colony forming unit (cfu). When a bacterium is contained in a bacterium composition, the "heating of a bacterium" may mean heating of the bacterium composition.

The survival rate, after heating at 70°C for 1 minute, of the bacterium that has been subjected to the operations of Steps A and B (more specifically, the survival rate of the bacterium after heating, at 70°C for 1 minute, of a bacterium composition obtained by the method of the disclosure) may be, for example, 10 or more times, 100 or more times, 1000 or more times, 10,000 or more times, or 100,000 or more times the survival rate, after heating at 70°C for 1 minute, of the bacterium before being subjected to the operations of Steps A and B.

The survival rate, after heating at 70°C for 1 minute, of the bacterium that has been subjected to the operations of Steps A and B (more specifically, the survival rate of the bacterium after heating, at 70°C for 1 minute, of a bacterium composition obtained by the method of the disclosure) may be, for example, 1% or more, 3% or more, 5% or more, or 10% or more.

The bacterium, the oil or fat, and the emulsifier are also collectively referred to as "effective components". In the method of the disclosure, the effective components may or may not be used alone. Thus, in the method of the disclosure, a component(s) other than the effective components may be additionally used. The components used in the method of the disclosure (that is, the effective components and the other arbitrary component(s)) are also collectively referred to as "raw materials".

More specifically, in Step A, a component(s) other than the bacterium and the oil or fat may be additionally used. In Step A, the term "raw materials" may be a collective term for the bacterium, the oil or fat, and the other arbitrary component(s). Further, more specifically, in Step B, a component(s) other than the bacterium and the emulsifier may be additionally used. In Step B, the term "raw materials" may be a collective term for the bacterium, the emulsifier, and the other arbitrary component(s).

<Bacterium>

The bacterium is not limited. As the bacterium, one bacterial species may be used, or two or more bacterial species may be used in combination. Examples of the bacterium include bacteria that can be used as probiotics. Specific examples of the bacterium include lactic acid bacteria. Examples of the lactic acid bacteria include bacteria belonging to the genus *Bifidobacterium,* bacteria belonging to the genus *Lactobacillus,* bacteria belonging to the genus *Lactococcus,* and bacteria belonging to the genus *Streptococcus.*

Examples of the bacteria belonging to the genus *Bifidobacterium* include *Bifidobacterium longum, Bifidobacterium breve, Bifidobacterium bifidum, Bifidobacterium adolescentis, Bifidobacterium angulatum, Bifidobacterium dentium, Bifidobacterium pseudocatenulatum, Bifidobacterium animalis, Bifidobacterium pseudolongum, and Bifidobacterium thermophilum.* Particular examples of the bacteria belonging to the genus *Bifidobacterium* include *Bifidobacterium longum* and *Bifidobacterium breve.* More particular examples of the bacteria belonging to the genus *Bifidobacterium* include *Bifidobacterium longum.*

The *Bifidobacterium longum* includes strains classified as any subspecies of *Bifidobacterium longum,* such as *B. longum* subsp. *longum, B. longum* subsp. *infantis,* and *B. longum* subsp. *suis.* The *Bifidobacterium animalis* includes strains classified as any subspecies of *Bifidobacterium animalis,* such as *B. animalis* subsp. *lactis.* The *Bifidobacterium pseudolongum* includes strains classified as any subspecies of *Bifidobacterium pseudolongum,* such as *B. pseudolongum* subsp. *globosum* and *B. pseudolongum* subsp. *pseudolongum).*

Specific examples of *Bifidobacterium longum* include BB536 (NITE BP-02621), ATCC 15697, ATCC 15707, ATCC 25962, ATCC 15702, ATCC 27533, M-63 (NITE BP-02623), BG7, DSM 24736, SBT 2928, NCC 490 (CNCM 1-2170), and NCC 2705 (CNCM 1-2618). Particular examples of *Bifidobacterium longum* include BB536. As *Bifidobacterium longum,* one strain may be used, or two or more strains may be used.

Specific examples of the *Bifidobacterium breve* include M-16V (NITE BP-02622), MCC1274 (FERM BP-11175), ATCC 15700, B632 (DSM 24706), Bb99 (DSM 13692), ATCC 15698, DSM 24732, UCC2003, YIT4010, YIT4064, BBG-001, BR-03, C50, and R0070. A particular example of *Bifidobacterium breve* includes M-16V. As *Bifidobacterium breve,* one strain may be used, or two or more strains may be used.

*Bifidobacterium longum* BB536 has been deposited with Patent Microorganisms Depositary, National Institute of Technology and Evaluation (Room 122, 2-5-8 Kazusakamatari, Kisarazu-shi, Chiba, 292-0818 Japan) under the accession No. NITE BP-02621 as of January 26, 2018 as an international deposit in accordance with the Budapest Treaty.

*Bifidobacterium breve* M-16V has been deposited with Patent Microorganisms Depositary, National Institute of Technology and Evaluation (Room 122, 2-5-8 Kazusakamatari, Kisarazu-shi, Chiba, 292-0818 Japan) under the accession No. NITE BP-02622 as of January 26, 2018 as an international deposit in accordance with the Budapest Treaty.

Specific examples of the *Bifidobacterium bifidum* include ATCC 29521, OLB6378, and BF-1. Specific examples of the *Bifidobacterium adolescentis* include ATCC 15703. Specific examples of *Bifidobacterium dentium* include DSM 20436. Specific examples of the *Bifidobacterium animalis* include DSM 10140, Bb-12, DN-173 010, GCL2505, and CNCM I-3446. Specific examples of *Bifidobacterium pseudolongum* include JCM 5820 and ATCC 25526. Specific examples of the *Bifidobacterium thermophilum* include ATCC 25525.

Examples of the bacteria belonging to the genus *Lactobacillus* include *Lactobacillus paracasei, Lactobacillus casei, Lactobacillus plantarum, Lactobacillus gasseri, Lactobacillus acidophilus,* and *Lactobacillus delbrueckii.* The *Lactobacillus delbrueckii* includes strains classified as any subspecies of *Lactobacillus delbrueckii,* such as *Lactobacillus delbrueckii* subsp. *bulgaricus, Lactobacillus delbrueckii* subsp. *delbrueckii,* and *Lactobacillus delbrueckii* subsp. *lactis.*

Examples of the bacteria belonging to the genus *Lactococcus* include *Lactococcus lactis.* The *Lactococcus lactis* includes strains classified as any subspecies of *Lactococcus lactis,* such as *Lactococcus lactis* subsp. *lactis, Lactococcus lactis* subsp. *cremoris,* and *Lactococcus lactis* subsp. *hordniae.*

Examples of the bacteria belonging to the genus *Streptococcus* include *Streptococcus thermophilus.*

These bacterial strains can be obtained from, for example, the American Type Culture Collection (ATCC, Address: 10801 University Boulevard Manassas, VA 20110, United States of America), Belgian Coordinated Collections of Microorganisms (BCCM, Address: Rue de la Science 8, 1000 Brussels, Belgium), German Collection of Microorganisms and Cell Cultures (DSMZ, Address: Inhoffenstr. 7B, D38124 Braunschweig, Germany), Japan Collection of Microorganisms (JCM, Microbe Division, RIKEN BioResource Research Center, 3-1-1 Koyadai, Tsukuba-shi, Ibaraki 305-0074, Japan), or a depository institution where each bacterial strain is deposited.

The bacterial strains specified by the bacterial strain names exemplified above are not limited to the strains themselves deposited or registered under those bacterial strain names in the predetermined institutions (hereinafter also referred to as "deposited strains" for convenience in the description), but also include strains substantially equivalent thereto (also referred to as "derived strains" or "induced strains"). More specifically, for example, *"Bifidobacterium longum* BB536" includes not only the strain deposited with the above-described depositary institution under the accession No. BB536, but also strains substantially equivalent thereto. For each bacterial strain, the "strain substantially equivalent to the deposited strain" means a strain which belongs to the same species as the deposited strain, whose identity of the nucleotide sequence of the 16S rRNA gene to the nucleotide sequence of the 16S rRNA gene of the deposited strain is preferably 99.86% or more, more preferably 99.93% or more, still more preferably 100%, and which preferably has the same bacteriological properties as the deposited strain. For each bacterial strain, the strain substantially equivalent to the deposited strain may be, for example, a derived strain whose parent strain is the deposited strain. Examples of the derived strain include strains bred from the deposited strain, and strains that have naturally occurred from the deposited strain. Examples of the breeding method include modification by a genetic engineering method, and modification by mutation treatment. Examples of the mutation treatment include X-ray irradiation, ultraviolet irradiation, and treatment with a mutagen such as N-methyl-N'-nitro-N-nitrosoguanidine (MNNG), ethyl methane sulfonate (EMS), or methyl methane sulfonate (MMS). Examples of the strains that have naturally occurred from the deposited strain include strains that have naturally occurred during use of the deposited strain. Examples of such strains include mutant strains that have naturally occurred by culture (for example, subculture) of the deposited strain. The derived strain may be constructed by a single modification, or may be constructed by two or more modifications.

As the bacterium, a commercially available product may be used, or a bacterium obtained by appropriate production may be used. Particular examples of commercially available products of the bacteria belonging to the genus *Bifidobacterium* include *Bifidobacterium longum* subsp. *longum* BB536 and *Bifidobacterium breve* M-16V, manufactured by Morinaga Milk Industry Co., Ltd. The bacterial cells can be easily obtained by culturing the bacterium. The culture method is not limited as long as the method allows for growth of the bacterium. Examples of the culture method include methods normally used for culture of bacteria such as bacteria belonging to the genus *Bifidobacterium,* which methods may be used as they are or after appropriate modification. The culture temperature may be, for example, 25 to 50°C. The culture temperature is preferably 35 to 42°C. The culture may be preferably carried out under anaerobic conditions. For example, the culture may be carried out under aeration with an anaerobic gas such as carbon dioxide gas. The culture may also be carried out under microaerobic conditions by, for example, static liquid culture. The culture may be carried out until, for example, the bacterium grows to a desired extent.

The medium used for the culture is not limited as long as the medium allows for growth of the bacterium. Examples of the medium include media normally used for culture of bacteria such as bacteria belonging to the genus *Bifidobacterium,* which media may be used as they are or after appropriate modification. The medium may contain, for example, a carbon source, a nitrogen source, an inorganic salt, an organic component, a milk component, or a combination thereof. Examples of the carbon source include saccharides such as galactose, glucose, fructose, mannose, cellobiose, maltose, lactose, sucrose, trehalose, starch, starch hydrolysate, and waste molasses, which may be used depending on the assimilability. Examples of the nitrogen source include ammonia, and ammonium salts and nitrates, such as ammonium sulfate, ammonium chloride, and ammonium nitrate. Examples of the inorganic salt include sodium chloride, potassium chloride, potassium phosphate, magnesium sulfate, calcium chloride, calcium nitrate, manganese chloride, and ferrous sulfate. Examples of the organic component include peptone, soy powder, defatted soybean meal, meat extract, and yeast extract. Examples of the milk component include milk protein. Examples of the milk protein include casein, whey, and degradation products thereof. Specific examples of media normally used for culture of bacteria such as bacteria belonging to the genus *Bifidobacterium* include reinforced clostridial medium, MRS medium (de Man, Rogosa, and Sharpe medium), mMRS medium (modified MRS medium), TOSP medium (TOS propionate medium), and TOSP Mup medium (TOS propionate mupirocin medium).

As the bacterium, bacterial cells or a fraction containing them may be used without limitation. More specifically, as the bacterium, a culture obtained by culturing may be used as it is. Alternatively, a diluted or concentrated culture may be used. Alternatively, bacterial cells collected from a culture may be used. As long as the heat resistance-improving effect is not deteriorated, the culturing may be followed by various additional operations such as heating, spray drying, or freeze-drying. The additional operations are preferably operations which give a high survival rate of the bacterial cells. Thus, specific examples of the bacterial cells or the fraction containing them include: bacterial cultures; bacterial cells collected from the cultures; and dilutions, concentrates, and dried products thereof.

The bacterial cells are used in a form containing viable bacterial cells. For example, the bacterial cells may be composed of viable bacterial cells, or may be a mixture of viable bacterial cells and dead bacterial cells.

From the viewpoint of the survival rate of the bacterial cells, a freeze-dried bacterial powder is preferably used as the bacterium. Thus, particular examples of the bacterium (more specifically, the bacterial cells or the fraction containing them) include freeze-dried bacterial powders. Before the freeze-drying, the bacterial cells are preferably dispersed in a dispersion medium together with a protecting agent and/or the like. More specifically, the bacterial cells may be collected from the culture and then washed to obtain wet bacterial cells, followed by dispersing the wet bacterial cells in water to prepare a bacterial cell dispersion. Subsequently, a protecting agent and/or the like may be preferably added to the bacterial cell dispersion, and the resulting mixture may be uniformly mixed, followed by freeze-drying the mixture by a normal method, to obtain a freeze-dried bacterial powder. Examples of the protecting agent that may be used include known protecting agents, such as amino acids including aspartic acid, arginine, glutamic acid, proline, lysine, leucine, and methionine; glycerin; sucrose; and ascorbic acid. The freeze-dried bacterial powder may be used as it is, or after mixing with other food materials. The freeze-dried bacterial powder is preferably used as it is.

### <Oil or Fat>

The oil or fat is not limited as long as the combined use with the emulsifier provides the heat resistance-improving effect. As the oil or fat, one kind of oil or fat may be used, or two or more kinds of oil(s) and/or fat(s) may be used in combination. As the oil or fat, a commercially available product may be used, or an oil or fat obtained by appropriate production may be used.

Examples of the oil or fat include oils and fats derived from animals (animal oils and fats), oils and fats derived from plants (vegetable oils and fats), and hydrogenated oils thereof. Examples of the animal oils and fats include chicken fat, lard, beef tallow, sheep fat, whale oil, fish oil, egg oil, and butter. Examples of the fish oil include tuna oil, bonito oil, sardine oil, mackerel oil, salmon oil, cod oil. Examples of the vegetable oils and fats include rapeseed oil, rice oil, safflower oil, sunflower oil, olive oil, peanut oil, palm oil, palm kernel oil, coconut oil, soybean oil, corn oil, cottonseed oil, sesame oil, grape seed oil, perilla oil. Examples of the hydrogenated oils include partially hydrogenated oils and fully hydrogenated oils. Specific examples of the hydrogenated oils include partially hydrogenated oils of the vegetable oils and fats exemplified above, and fully hydrogenated oils of the vegetable oils and fats exemplified above. Particular examples of the hydrogenated oils include hydrogenated oils of palm kernel oil. More particular examples of the hydrogenated oils include fully hydrogenated oils of palm kernel oil (fully hydrogenated palm kernel oils). Examples of commercially available fully hydrogenated palm kernel oils include K-40 (manufactured by Taiyo Yushi Corp.)

Examples of the oil or fat include those having a melting point within a particular range. As the oil or fat, for example, an oil or fat having a melting point within a particular range may be selected from the oils and fats exemplified above. The fully hydrogenated palm kernel oil may have a melting point of about 40°C. The melting point of the oil or fat may be, for example, 20°C or more, 25°C or more, 30°C or more, 35°C or more, 40°C or more, 45°C or more, or 50°C or more, and may be, for example, 55°C or less, 50°C or less, 45°C or less, 40°C or less, 35°C or less, 30°C or less, or 25°C or less. The melting point may be within a range consistently defined by their combination. More specifically, the melting point of the oil or fat may be, for example, 20 to 55°C, 25 to 50°C, 30 to 50°C, 35 to 50°C, or 40 to 50°C. More specifically, the melting point of the oil or fat may be, for example, 20 to 25°C, 25 to 30°C, 30 to 35°C, 35 to 40°C, 40 to 45°C, 45 to 50°C, or 50 to 55°C. In particular, the melting point of the oil or fat may be 35°C or more, may be 50°C or less, or may be 35 to 50°C.

### <Emulsifier>

The emulsifier is not limited as long as the combined use with the oil or fat provides the heat resistance-improving effect. As the emulsifier, one kind of emulsifier may be used, or two or more kinds of emulsifiers may be used in combination. As the emulsifier, a commercially available product may be used, or an emulsifier obtained by an appropriate production method may be used.

Examples of the emulsifier include glycerin fatty acid esters, sucrose fatty acid esters, propylene glycol fatty acid esters, lecithins, and saponins. Examples of the glycerin fatty acid esters include monoglycerin fatty acid esters and polyglycerin fatty acid esters. Particular examples of the glycerin fatty acid esters include polyglycerin fatty acid esters. Particular examples of the emulsifier include polyglycerin fatty acid esters and lecithins. More particular examples of the emulsifier include lecithins.

The "polyglycerin fatty acid ester" is a general term for compounds having a structure containing a fatty acid bound to a hydroxyl group of a polyglycerin via an ester bond. The molecular structure of the polyglycerin fatty acid ester (for example, the degree of polymerization of the polyglycerin, the polymerization mode of the polyglycerin (linear, circular, or branched), the type of the constituent fatty acid, the esterification rate, and the esterification position) is not limited as long as the combined use with the oil or fat provides the heat resistance-improving effect. Examples of the constituent fatty acid include fatty acids having 8 to 24 carbon atoms. The constituent fatty acid may be either a saturated fatty acid or an unsaturated fatty acid. Specific examples of the fatty acids having 8 to 24 carbon atoms include caprylic acid, lauric acid, myristic acid, palmitic acid, stearic acid, oleic acid, behenic acid, and erucic acid. As the constituent fatty acid, one kind of fatty acid may be used, or two or more kinds of fatty acids may be used in combination. For example, two or more kinds of fatty acids may be bound to one molecule of polyglycerin through ester bonds.

The degree of polymerization (average degree of polymerization) of the polyglycerin may be, for example, 2 or more, 3 or more, 4 or more, 5 or more, 6 or more, 7 or more, or 8 or more, and may be, for example, 20 or less, 15 or less, 12 or less, 10 or less, 8 or less, or 5 or less. The degree of polymerization may be within a range consistently defined by their combination. More specifically, the degree of polymerization (average degree of polymerization) of the polyglycerin is, for example, preferably 2 to 20, 3 to 15, 3 to 12, or 3 to 5. The degree of polymerization (average degree of polymerization) of the polyglycerin is calculated based on the hydroxyl value of the polyglycerin. The hydroxyl value of the polyglycerin is measured according to the Japanese Industrial Standard JIS K 0070: 1992.

The HLB (hydrophile-lipophile balance) value of the polyglycerin may be, for example, 1 or more, 2 or more, 3 or more, or 4 or more, and may be, for example, 20 or less, 15 or less, 10 or less, 8 or less, or 5 or less. The HLB value may be within a range consistently defined by their combination. More specifically, the HLB value of the polyglycerin is, for example, preferably 1 to 15, 1 to 10, 2 to 8, or 2 to 5. The HLB value is calculated by the Atlas method.

The description on the molecular structure of the polyglycerin fatty acid ester (such as the type of the constituent fatty acid) can also apply to other fatty acid esters.

Examples of commercially available polyglycerin fatty acid esters include POEM RJ-38 (manufactured by Riken Vitamin Co., Ltd.) POEM RJ-38 is a polyglycerin fatty acid ester containing C18 (65%) and C16 (34%) constituent fatty acids.

"Lecithin" is a general term for materials containing phospholipid. The lecithin may contain phospholipid as a major component. Examples of the lecithin include vegetable lecithin, yolk lecithin, and derivatives thereof. Examples of the vegetable lecithin include soybean lecithin, rape lecithin, and sunflower lecithin. Examples of the derivatives include fractionated lecithin, enzymetreated lecithin, and enzyme-degraded lecithin. A particular example of the lecithin is soybean lecithin.

The HLB value of the lecithin may be, for example, 1 or more, 2 or more, 3 or more, or 4 or more, and may be, for example, 20 or less, 15 or less, 10 or less, 8 or less, or 5 or less. The HLB value may be within a range consistently defined by their combination. More specifically, the HLB value of the lecithin is, for example, preferably 1 to 15, 1 to 10, 2 to 8, or 2 to 5.

Examples of commercially available lecithins include Yelkin TS (HLB value = about 4, manufactured by ADM Japan Ltd.) Yelkin TS is a soybean lecithin in a liquid form.

"Saponin" is a general term for sapogenin glycosides. Examples of the saponin include saponins of various plants. Specific examples of the saponin include soybean saponin, tea seed saponin, and sophora saponin.

### <Other Component(s)>

The other component(s) is/are not limited as long as the heat resistance-improving effect is not deteriorated. As the other component(s), one component may be used, or two or more components may be used in combination.

As the other component(s), components acceptable depending on the mode of use of the bacterium composition may be used. Examples of the other component(s) include orally ingestible components. Examples of the orally ingestible components include components used as a mixture with a food or beverage, or with a pharmaceutical.

### <Steps A and B>

Step A is a step of mixing the bacterium with the oil or fat. Step B is a step of mixing the bacterium with the emulsifier. The bacterium is subjected to both Steps A and B (that is, the mixing with the oil or fat and the mixing with the emulsifier).

Steps A and B may be carried out at the same time, or may be carried out separately. In particular, Steps A and B may be carried out separately. The order of carrying out Steps A and B is not limited. Thus, Step B may be carried out after carrying out Step A, or Step A may be carried out after carrying out Step B. In particular, Step B may be carried out after carrying out Step A. The term "Steps A and B are carried out at the same time" means that, before completion of one of Steps A and B, the other of Steps A and B is begun. The term "Steps A and B are carried out at the same time" includes when Steps A and B are begun at the same time, and also when, during the operation of one of Steps A and B, the other of Steps A and B is begun. Thus, the term "Steps A and B are carried out at the same time" may mean that Steps A and B are carried out at the same time during part of or throughout the period. When Steps A and B are carried out at the same time, Steps A and B may be completed at the same time, or may be completed separately. The term "Steps A and B are carried out separately" means that, after the completion of one of Steps A and B, the other of Steps A and B is begun. The term "during a step" means the period from the beginning to the completion of the step. The term "completion of a step" may mean a state where the step has been sufficiently carried out, more specifically, where the step has been carried out for a predetermined period of time. The "predetermined period of time" during which each step is carried out is, for example, the operation time of each step described later.

In other words, Steps A and B may begin at the same time, or may begin at different times. In particular, Steps A and B may begin at different times. Thus, after the beginning of one of Steps A and B, the other of Steps A and B may begin. More specifically, during the operation or after the completion of one of Steps A and B, the other of Steps A and B may begin. The order of beginning of Steps A and B is not limited. Thus, Step B may begin after the beginning of Step A, or Step A may begin after the beginning of Step B. In particular, Step B may begin after the beginning of Step A. Furthermore, in particular, Step B may begin after the completion of Step A.

By carrying out Step A, for example, the bacterium may be coated with the oil or fat. By carrying out Step B, for example, the bacterium may be coated with the emulsifier. By carrying out Steps A and B, for example, the bacterium may be coated with the oil or fat and the emulsifier. More specifically, by carrying out Steps A and B, for example, the bacterium may be coated with a mixture of the oil or fat and the emulsifier. Alternatively, more specifically, by carrying out Steps A and B, for example, the bacterium may be coated with the oil or fat, and further coated with the emulsifier. In particular, when Step B begins after Step A, the bacterium may be coated with the oil or fat, and further coated with the emulsifier. Alternatively, more specifically, by carrying out Steps A and B, for example, the bacterium may be coated with the emulsifier, and further coated with the oil or fat. In particular, when Step A begins after Step B, the bacterium may be coated with the emulsifier, and further coated with the oil or fat. Thus, unless otherwise specified, the term "bacterium is coated with the oil or fat and the emulsifier" may mean that the bacterium is coated with the oil or fat and the emulsifier irrespective of the mixing state of the oil or fat and the emulsifier, and irrespective of the order of coating with the oil or fat and the emulsifier.

The term "bacterium is coated with the oil or fat" includes not only when the bacterium is coated with the oil or fat alone, but also when the bacterium is coated with a mixture of the oil or fat and one or more other components. For example, when Step A is carried out, when one or more other components coexist with the bacterium and the oil or fat, the bacterium may be coated with a mixture of the oil or fat and the other component(s).

The term "bacterium is coated with the emulsifier" includes not only when the bacterium is coated with the emulsifier alone, but also when the bacterium is coated with a mixture of the emulsifier and one or more other components. For example, when Step B is carried out, when one or more other components coexist with the bacterium and the emulsifier, the bacterium may be coated with a mixture of the emulsifier and the other component(s).

When Step B begins after Step A, the term "bacterium" subjected to Step B may be read as the bacterium as it exists after the beginning of Step A. The bacterium as it exists after the beginning of Step A may be, for example, a mixture of the bacterium and the oil or fat. More specifically, the bacterium after the beginning of Step A may be, for example, a bacterium coated with the oil or fat. When Step A begins after Step B, the term "bacterium" subjected to Step A may be read as the bacterium after the beginning of Step B. The bacterium after the beginning of Step B may be, for example, a mixture of the bacterium and the emulsifier. More specifically, the bacterium after the beginning of Step B may be, for example, a bacterium coated with the emulsifier.

In Step A, any of the raw materials (the bacterium and the oil or fat, and the other arbitrary component(s)) may be used in an arbitrary form such as a liquid, paste or powder. In particular, for example, the oil or fat may be used in the form of a liquid for Step A. The oil or fat in the liquid form may be naturally in the liquid form, or may be an oil or fat prepared by treatment (for example, heating) such that it is in the liquid form. Thus, the oil or fat may be preliminarily heated to the melting point or a higher temperature before the use in Step A. For carrying out Step A, for example, the oil or fat may be added to the bacterium, or the bacterium may be added to the oil or fat. The means of the addition may be appropriately set depending on conditions such as the form of the raw materials. For example, when a component to be added is in a liquid form, the component may be added dropwise or sprayed. More specifically, for example, when the oil or fat is in a liquid form, the oil or fat may be added dropwise or sprayed onto the bacterium. When another component is used, the other component may or may not be preliminarily mixed with the bacterium and/or the oil or fat.

In Step B, any of the raw materials (the bacterium and the emulsifier, and the other arbitrary component(s)) may be used in an arbitrary form such as a liquid, paste or powder. In particular, for example, the emulsifier may be used in the form of a liquid for Step B. The emulsifier in the liquid form may be naturally in the liquid form, or may be an emulsifier prepared by treatment (for example, heating) such that it is in the liquid form. Thus, the emulsifier may be preliminarily heated to the melting point or a higher temperature before the use in Step B. For carrying out Step B, for example, the emulsifier may be added to the bacterium, or the bacterium may be added to the emulsifier. The means of the addition may be appropriately set depending on conditions such as the forms of the raw materials. For example, when a component to be added is in a liquid form, the component may be added dropwise or sprayed. More specifically, for example, when the emulsifier is in a liquid form, the emulsifier may be added dropwise or sprayed onto the bacterium. When another component is used, the other component may or may not be preliminarily mixed with the bacterium and/or the emulsifier.

When Steps A and B are carried out at the same time, the oil or fat and the emulsifier may be used, for example, separately or as a mixture.

The means of mixing the raw materials is not limited. Examples of the means of mixing the raw materials include mixing by stirring, and mixing by inversion. Particular examples of the means of mixing the raw materials include mixing by stirring. The raw materials may be mixed, for example, in an appropriate container. The container in which the raw materials are mixed is also referred to as "mixing container". Steps A and B may or may not be carried out in the same mixing container. Each of Steps A and B may be carried out by, for example, either a batch method or a continuous method. The raw materials may be mixed, for example, using a mixing apparatus. The mixing apparatus may be equipped with, for example, a mixing container. Further, the mixing apparatus may be equipped with, for example, a stirring blade. The stirring blade is also called an agitator or a chopper, for example. Furthermore, the mixing apparatus may have, for example, a temperature control function. Examples of the mixing apparatus include a stirring granulator. Specific examples of the mixing apparatus include a high-speed stirring-type mixing granulator (such as the NMG series, manufactured by Nara Machinery Co., Ltd.), New Speed Kneader (such as the NSK series, manufactured by Okada Seiko Co., Ltd.), BALANCE GRAN (such as the BG series, manufactured by Freund-Turbo Corporation), FLEXOMIX (such as FXD-250, manufactured by Hosokawa Micron Corporation), and Vertical Granulator (manufactured by Powrex Corporation).

The ratios of the amounts of the raw materials used in the method of the disclosure are not limited as long as the heat resistance-improving effect can be obtained. The ratios of the amounts of the raw materials used in the method of the disclosure may be appropriately set depending on conditions such as the types of the raw materials, the form (dosage form) of the bacterium composition, and the intended use of the bacterium composition.

The amount of the oil or fat used per 50 g dry cell weight of the bacterium may be, for example, 1.2 g or more, 1.5 g or more, 2 g or more, 2.5 g or more, 3 g or more, or 3.5 g or more, and may be, for example, 4.8 g or less, 4.5 g or less, 4 g or less, 3.5 g or less, 3 g or less, or 2.5 g or less. The amount may be within a range consistently defined by their combination. More specifically, the amount of the oil or fat used per 50 g dry cell weight of the bacterium may be, for example, 1.2 to 4.8 g, preferably 1.5 to 4.5 g, more preferably 2 to 4 g. The amount of the oil or fat may be set using the viable cell count of the bacterium as a standard. In such a case, the term "per 50 g dry cell weight of the bacterium" may be read as, for example, "per 4×10¹² cfu viable cells of the bacterium".

The amount of the emulsifier used per 50 g dry cell weight of the bacterium may be, for example, 1 g or more, 1.5 g or more, 2 g or more, 3 g or more, 4 g or more, 5 g or more, 6 g or more, 7 g or more, 8 g or more, 9 g or more, or 10 g or more, and may be, for example, 20 g or less, 17 g or less, 15 g or less, 14 g or less, 13 g or less, 12 g or less, 11 g or less, 10 g or less, 9 g or less, or 8 g or less. The amount may be within a range consistently defined by their combination. More specifically, the amount of the emulsifier used per 50 g dry cell weight of the bacterium may be, for example, 1 to 20 g, 1.5 to 17 g, or 2 to 15 g, or may be, for example, 8 to 17 g, 9 to 16 g, or 10 to 15 g. In particular, when the emulsifier is a lecithin, the amount of the emulsifier used per 50 g dry cell weight of the bacterium may be 1 to 20 g, preferably 1.5 to 17 g, more preferably 2 to 15 g. In particular, when the emulsifier is a polyglycerin fatty acid ester, the amount of the emulsifier used per 50 g dry cell weight of the bacterium may be 8 to 17 g, preferably 9 to 16 g, more preferably 10 to 15 g. Furthermore, in particular, when the emulsifier is a lecithin, the amount of the emulsifier used per 50 g dry cell weight of the bacterium may be half the amount exemplified above in terms of the weight of phospholipid. The amount of the emulsifier used may be set using the viable cell count of the bacterium as a standard. In such a case, the term "per 50 g dry cell weight of the bacterium" may be read as, for example, "per 4×10¹² cfu viable cells of the bacterium".

The total amount of the effective components used, in terms of the weight ratio to the total amount of the raw materials used, may be, for example, 1% or more, 3% or more, 5% or more, 10% or more, 20% or more, 30% or more, 40% or more, 50% or more, 60% or more, 70% or more, 80% or more, 90% or more, 95% or more, 97% or more, or 99% or more, and may be, for example, 100% or less, 99% or less, 97% or less, 95% or less, 90% or less, 80% or less, 70% or less, 60% or less, or 50% or less. The total amount may be within a range consistently defined by their combination. More specifically, the total amount of the effective components used, in terms of the weight ratio to the total amount of the raw materials used, may be, for example, 1 to 100%, 10 to 100%, 30 to 100%, 50 to 100%, 70 to 100%, 90 to 100%, 95 to 100%, or 99 to 100%.

The operation time of Step A is not limited as long as the heat resistance-improving effect can be obtained. The operation time of Step A may be appropriately set depending on conditions such as the types of the raw materials, the form (dosage form) of the bacterium composition, and the intended use of the bacterium composition. The operation time of Step A may be, for example, 30 seconds or more, 45 seconds or more, 1 minute or more, 1.5 minutes or more, 2 minutes or more, 2.5 minutes or more, 3 minutes or more, 3.5 minutes or more, or 4 minutes or more, and may be, for example, 15 minutes or less, 10 minutes or less, 7 minutes or less, 5 minutes or less, 4.5 minutes or less, 4 minutes or less, 3.5 minutes or less, 3 minutes or less, or 2.5 minutes or less. The period of time may be within a range consistently defined by their combination. More specifically, the operation time of Step A may be, for example, 30 seconds to 15 minutes, preferably 45 seconds to 10 minutes, more preferably 1 minute to 7 minutes.

The operation time of Step B is not limited as long as the heat resistance-improving effect can be obtained. The operation time of Step B may be appropriately set depending on conditions such as the types of the raw materials, the form (dosage form) of the bacterium composition, and the intended use of the bacterium composition. The operation time of Step B may be, for example, 30 seconds or more, 1 minute or more, 2 minutes or more, 3 minutes or more, 5 minutes or more, 7 minutes or more, 10 minutes or more, 15 minutes or more, 20 minutes or more, 25 minutes or more, or 30 minutes or more, and may be, for example, 180 minutes or less, 150 minutes or less, 120 minutes or less, 100 minutes or less, 90 minutes or less, 80 minutes or less, 70 minutes or less, 60 minutes or less, 50 minutes or less, 45 minutes or less, 40 minutes or less, 35 minutes or less, 30 minutes or less, 25 minutes or less, 20 minutes or less, 15 minutes or less, or 10 minutes or less. The period of time may be within a range consistently defined by their combination. More specifically, the operation time of Step B may be, for example, 30 seconds to 30 minutes, 1 minute to 20 minutes, or 2 minutes to 15 minutes, or may be, for example, 30 seconds to 180 minute, 1 minute to 120 minutes, or 2 minutes to 90 minutes. In particular, when the emulsifier is a polyglycerin fatty acid ester, the operation time of Step B may be 30 seconds to 40 minutes, preferably 1 minute to 30 minutes, more preferably 2 minutes to 20 minutes. In particular, when the emulsifier is a lecithin, the operation time of Step B may be 30 seconds to 180 minutes, preferably 1 minute to 120 minutes, more preferably 2 minutes to 90 minutes.

When Step B is begun after Step A, for example, Step B may be begun after the beginning of Step A, but before the end of the operation time of Step A exemplified above. Alternatively, Step B may be begun after the end of the operation time of Step A exemplified above. For example, the period of time from the beginning of Step A to the beginning of Step B may be set to the operation time of Step A exemplified above.

When Step A begins after Step B, for example, Step A may begin after the beginning of Step B, but before the end of the operation time of Step B exemplified above. Alternatively, Step A may begin after the end of the operation time of Step B exemplified above. For example, the period of time from the beginning of Step B to the beginning of Step A may be set to the operation time of Step B exemplified above.

The total operation time of Steps A and B may be set such that, for example, the operation times exemplified above for these steps can be secured. The term "total operation time of Steps A and B" means the period of time from the beginning of the step, either A or B, whichever is begun earlier, to the completion of the step, either A or B, whichever is completed later. When both steps are begun at the same time, the term "beginning of the step, either A or B, whichever is begun earlier" means the beginning of both steps. When both steps are completed at the same time, the term "completion of the step, either A or B, whichever is completed later" means the completion of both steps. When both Steps A and B begin at the same time, the total operation time of Steps A and B may be set to, for example, the longer operation time between the operation time of Step A exemplified above and the operation time of Step B exemplified above.

The operation conditions (such as the temperature and/or the mixing rate) for Steps A and B may be either the same or different. At least during the period when Steps A and B are carried out at the same time, the operation conditions for Steps A and B are the same.

The operation conditions (such as the temperature and/or the mixing rate) for Step A may or may not be constant throughout the period of the operation of Step A.

The operation conditions (such as the temperature and/or the mixing rate) for Step B may or may not be constant throughout the period of the operation of Step B.

Step A is carried out at the melting point of the oil or fat or a higher temperature. Thus, the temperature in Step A (that is, the temperature at which Step A is carried out) is the melting point of the oil or fat or a higher temperature. The temperature in Step A is not limited as long as it is the melting point of the oil or fat or a higher temperature, and as long as the heat resistance-improving effect can be obtained. The temperature in Step A may be appropriately set depending on conditions such as the types of the raw materials, the form (dosage form) of the bacterium composition, and the intended use of the bacterium composition. The temperature in Step A may be higher than the melting point of the oil or fat by, for example, 1°C or more, 2°C or more, 3°C or more, 4°C or more, or 5°C or more. The temperature in Step A may be, for example, 20°C or more, 25°C or more, 30°C or more, 35°C or more, 40°C or more, 45°C or more, or 50°C or more, and may be, for example, 55°C or less, 50°C or less, 45°C or less, 40°C or less, 35°C or less, 30°C or less, or 25°C or less. The temperature may be within a range consistently defined by their combination. More specifically, the temperature in Step A may be, for example, 20 to 55°C, 25 to 50°C, 30 to 50°C, 35 to 50°C, or 40 to 50°C. More specifically, the temperature in Step A may be, for example, 20 to 25°C, 25 to 30°C, 30 to 35°C, 35 to 40°C, 40 to 45°C, 45 to 50°C, or 50 to 55°C. In particular, the temperature in Step A may be 40°C or more, may be 50°C or less, or may be 40 to 50°C. The temperature may be usually within the temperature range exemplified above throughout the period of Step A. However, the temperature may be temporarily outside such a range. Thus, the term "temperature in Step A is within a certain range" includes not only when the temperature is within the range throughout the period of Step A, but also when the temperature is temporarily outside the range. For example, in Step A, the temperature may be temporarily below the melting point of the oil or fat. The length meant by the term "temporarily" for Step A is not limited as long as the heat resistance-improving effect can be obtained. The term "temporarily" for Step A may mean a period corresponding to, for example, 20% or less, 15% or less, 10% or less, 5% or less, 3% or less, or 1% or less of the entire period of Step A. The temperature in Step A is set such that the bacterium survives to a desired extent in the operation of Step A. The survival rate of the bacterium in the operation of Step A may be, for example, 50% or more, 70% or more, 90% or more, or 95% or more. The survival rate of the bacterium in the operation of Step A is calculated as the ratio of the viable cell count after the operation of Step A (more specifically, at the completion of Step A) to the viable cell count before the operation of Step A (more specifically, at the beginning of Step A).

The temperature in Step B (that is, the temperature at which Step B is carried out) is not limited as long as the heat resistance-improving effect can be obtained. The temperature in Step B may be appropriately set depending on conditions such as the types of the raw materials, the form (dosage form) of the bacterium composition, and the intended use of the bacterium composition. Step B may be carried out at the melting point of the emulsifier or a higher temperature. Thus, the temperature in Step B may be, for example, the melting point of the emulsifier or a higher temperature. The temperature in Step B may be higher than the melting point of the emulsifier by, for example, 1°C or more, 2°C or more, 3°C or more, 4°C or more, or 5°C or more. The temperature in Step B may be, for example, 20°C or more, 25°C or more, 30°C or more, 35°C or more, 40°C or more, 45°C or more, or 50°C or more, and may be, for example, 55°C or less, 50°C or less, 45°C or less, 40°C or less, 35°C or less, 30°C or less, or 25°C or less. The temperature may be within a range consistently defined by their combination. More specifically, the temperature in Step B may be, for example, 20 to 55°C, 25 to 50°C, 30 to 50°C, 35 to 50°C, or 40 to 50°C. More specifically, the temperature in Step B may be, for example, 20 to 25°C, 25 to 30°C, 30 to 35°C, 35 to 40°C, 40 to 45°C, 45 to 50°C, or 50 to 55°C. In particular, the temperature in Step B may be 40°C or more, may be 50°C or less, or may be 40 to 50°C. The temperature may be usually within the temperature range exemplified above throughout the period of Step B. However, the temperature may be temporarily outside such a range. Thus, the term "temperature in Step B is within a certain range" includes not only when the temperature is within the range throughout the period of Step B, but also when the temperature is temporarily outside the range. The length meant by the term "temporarily" for Step B is not limited as long as the heat resistance-improving effect can be obtained. The term "temporarily" for Step B may mean a period corresponding to, for example, 20% or less, 15% or less, 10% or less, 5% or less, 3% or less, or 1% or less of the entire period of Step B. The temperature in Step B is set such that the bacterium survives to a desired extent in the operation of Step B. The survival rate of the bacterium in the operation of Step B may be, for example, 50% or more, 70% or more, 90% or more, or 95% or more. The survival rate of the bacterium in the operation of Step B is calculated as the ratio of the viable cell count after the operation of Step B (more specifically, at the completion of Step B) to the viable cell count before the operation of Step B (more specifically, at the beginning of Step B).

The temperatures of both Steps A and B may be appropriately controlled (for example, increased) such that they are within the temperature ranges exemplified above. The method of controlling (for example, increasing) the temperatures is not limited. For example, the mixing container may be heated to control (for example, to increase) the temperature. The means of heating the mixing container is not limited as long as the inside of the mixing container can be heated (more specifically, the raw materials inside the mixing container can be heated) therein. The heating means may be, for example, incorporated in the mixing container, or equipped outside the mixing container. Examples of the heating means equipped outside the mixing container include heating jackets. Thus, for example, the mixing container may be covered with a heating jacket, and the jacket may be heated to heat the inside of the mixing container from the outside.

The mixing rate in Step A is not limited as long as the heat resistance-improving effect can be obtained. The mixing rate in Step A may be appropriately set depending on conditions such as the types of the raw materials, the form (dosage form) of the bacterium composition, the intended use of the bacterium composition, and the type of the mixing apparatus employed. The mixing rate in Step A, in terms of the rotation speed of the stirring blade, may be, for example, 100 rpm or more, 300 rpm or more, 500 rpm or more, 1000 rpm or more, 1500 rpm or more, 2000 rpm or more, or 2500 rpm or more, and may be, for example, 5000 rpm or less, 4000 rpm or less, 3000 rpm or less, 2500 rpm or less, 2000 rpm or less, 1500 rpm or less, or 1000 rpm or less. The mixing rate may be within a range consistently defined by their combination. More specifically, the mixing rate in Step A, in terms of the rotation speed of the stirring blade, may be, for example, 500 to 5000 rpm, preferably 1000 to 4000 rpm, more preferably 2000 to 3000 rpm. The mixing rate in Step A, in terms of the peripheral speed of the stirring blade (that is, the rotation velocity of the tip of the stirring blade), may be, for example, 1 m/s or more, 3 m/s or more, 5 m/s or more, 10 m/s or more, 15 m/s or more, 20 m/s or more, or 25 m/s or more, and may be, for example, 50 m/s or less, 40 m/s or less, 30 m/s or less, 25 m/s or less, 20 m/s or less, 15 m/s or less, or 10 m/s or less. The mixing rate may be within a range consistently defined by their combination. More specifically, the mixing rate in Step A, in terms of the peripheral speed of the stirring blade (that is, the rotation velocity of the tip of the stirring blade), may be, for example, 3 to 50 m/s, preferably 5 to 40 m/s, more preferably 10 to 30 m/s. The mixing rate may be usually within the mixing rate range exemplified above throughout the period of Step A. However, the mixing rate may be temporarily outside such a range. Thus, the term "mixing rate in Step A is within a certain range" includes not only when the mixing rate is within the range throughout the period of Step A, but also when the mixing rate is temporarily outside the range. For example, in Step A, the mixing of the raw materials may be temporarily stopped. Thus, in Step A, the mixing of the raw materials may be carried out either continuously or intermittently. The length meant by the term "temporarily" for Step A is not limited as long as the heat resistance-improving effect can be obtained. The term "temporarily" for Step A may mean a period corresponding to, for example, 20% or less, 15% or less, 10% or less, 5% or less, 3% or less, or 1% or less of the entire period of Step A.

The mixing rate in Step B is not limited as long as the heat resistance-improving effect can be obtained. The mixing rate in Step B may be appropriately set depending on conditions such as the types of the raw materials, the form (dosage form) of the bacterium composition, the intended use of the bacterium composition, and the type of the mixing apparatus employed. The mixing rate in Step B, in terms of the rotation rate of the stirring blade, may be, for example, 100 rpm or more, 300 rpm or more, 500 rpm or more, 1000 rpm or more, 1500 rpm or more, 2000 rpm or more, or 2500 rpm or more, and may be, for example, 5000 rpm or less, 4000 rpm or less, 3000 rpm or less, 2500 rpm or less, 2000 rpm or less, 1500 rpm or less, or 1000 rpm or less. The mixing rate may be within a range consistently defined by their combination. More specifically, the mixing rate in Step B, in terms of the rotation speed of the stirring blade, may be, for example, 500 to 5000 rpm, preferably 1000 to 4000 rpm, more preferably 2000 to 3000 rpm. The mixing rate in Step B, in terms of the peripheral speed of the stirring blade (that is, the rotation velocity of the tip of the stirring blade), may be, for example, 1 m/s or more, 3 m/s or more, 5 m/s or more, 10 m/s or more, 15 m/s or more, 20 m/s or more, or 25 m/s or more, and may be, for example, 50 m/s or less, 40 m/s or less, 30 m/s or less, 25 m/s or less, 20 m/s or less, 15 m/s or less, or 10 m/s or less. The mixing rate may be within a range consistently defined by their combination. More specifically, the mixing rate in Step B, in terms of the peripheral speed of the stirring blade (that is, the rotation velocity of the tip of the stirring blade), may be, for example, 3 to 50 m/s, preferably 5 to 40 m/s, more preferably 10 to 30 m/s. The mixing rate may be usually within the mixing rate range exemplified above throughout the period of Step B. However, the mixing rate may also be temporarily outside such a range. Thus, the term "mixing rate in Step B is within a certain range" includes not only when the mixing rate is within the range throughout the period of Step B, but also when the mixing rate is temporarily outside the range. For example, in Step B, the mixing of the raw materials may be temporarily stopped. Thus, in Step B, the mixing of the raw materials may be carried out either continuously or intermittently. The length meant by the term "temporarily" for Step B is not limited as long as the heat resistance-improving effect can be obtained. The term "temporarily" for Step B may mean a period corresponding to, for example, 20% or less, 15% or less, 10% or less, 5% or less, 3% or less, or 1% or less of the entire period of Step B.

By carrying out Steps A and B as described above, heat resistance of the bacterium can be improved. The bacterium having improved heat resistance may be obtained as a bacterium composition (more specifically, a heatresistant bacterium composition).

The bacterium composition may be obtained, for example, as a paste-like composition. The bacterium composition may be used as it is or after appropriate processing. The bacterium composition may be used, for example, after processing into an arbitrary form.

The use of the bacterium composition is not limited. The bacterium composition may be used by, for example, addition to a food or beverage. Thus, the bacterium composition may be a composition for addition to a food or beverage (that is, a composition used by addition to a food or beverage). The disclosure also provides a food or beverage containing the bacterium composition. The food or beverage is not limited. Examples of the food or beverage include dairy products. The dairy products may be products produced using cow milk as a raw material. Examples of the dairy products include powdered milks. Examples of the powdered milks include whole milk powder, skimmed milk powder, modified milk powder, powdered whey, and creaming powder. Specific examples of the foods and beverages such as powdered milks include those for babies and infants. Particular examples of the foods and beverages such as powdered milks include those for babies. Specific examples of the powdered milks include powdered formula milks for childcare, such as powdered baby formula milks and followup milks. The term "baby" means a child who is younger than 1 year old. The term "infant" means a child who is 1 year old or older but not yet attending school.

The content of the bacterium composition in the food or beverage is not limited. The content of the bacterium composition in the food or beverage may be, for example, 0.01% (w/w) or more, 0.1% (w/w) or more, 0.5% (w/w) or more, 1% (w/w) or more, 5% (w/w) or more, or 10% (w/w) or more, and may be, for example, 50% (w/w) or less, 30% (w/w) or less, 20% (w/w) or less, 10% (w/w) or less, or 5% (w/w) or less. The content may be within a range consistently defined by their combination. More specifically, the content of the bacterium composition in the food or beverage may be, for example, 0.01% (w/w) to 50% (w/w), 0.1% (w/w) to 50% (w/w), 0.1 (w/w) to 10 (w/w)%, or 0.5 (w/w) to 5 (w/w)%. The content of the bacterium composition in the food or beverage in terms of the content of the bacterium may be, for example, 1×10⁴ cfu/g or more, 1×10⁵ cfu/g or more, 1×10⁶ cfu/g or more, or 1×10⁷ cfu/g or more, and may be, for example, 1×10¹³ cfu/g or less, 1×10¹² cfu/g or less, 1×10¹¹ cfu/g or less, or 1×10¹⁰ cfu/g or less. The content may be within a range defined by their combination. More specifically, the content of the bacterium composition in the food or beverage in terms of the content of the bacterium may be, for example, 1×10⁵ to 1×10¹² cfu/g, preferably 1×10⁶ to 1×10¹¹ cfu/g, more preferably 1×10⁷ to 1×10¹⁰ cfu/g.

### <2> Composition

The composition of the disclosure is a composition containing a bacterium, an oil or fat, and an emulsifier. The bacterium, oil or fat, and emulsifier are also collectively referred to as "effective components".

The composition of the disclosure may or may not be composed of the effective components alone. Thus, the composition of the disclosure may contain another component in addition to the effective components.

To the components contained in the composition (that is, the effective components and other arbitrary component(s)), the description on the raw materials in the method of the disclosure may be applied. The bacterium contained in the composition of the disclosure may be a bacterium having improved heat resistance compared to those not contained in the composition of the disclosure. Thus, the composition of the disclosure may be a heat-resistant bacterium composition (a composition containing a bacterium having improved heat resistance). In the composition of the disclosure, the effect which improves heat resistance of the bacterium, which effect is obtained by including the bacterium in the composition of the disclosure, is also referred to as "heat resistance-improving effect". To the heat resistance-improving effect in the composition of the disclosure, the description regarding the heat resistance-improving effect in the method of the disclosure may be applied. Thus, the bacterium before the operations of Steps A and B in the method of the disclosure may be read as the bacterium not included in the composition of the disclosure. Furthermore, the bacterium after the operations of Steps A and B in the method of the disclosure may be read as the bacterium included in the composition of the disclosure.

The amounts, and the ratios of the amounts, of the components contained in the composition of the disclosure are not limited as long as the heat resistance-improving effect can be obtained. To the amounts, and the ratios of the amounts, of the components contained in the composition of the disclosure, the description regarding the amounts of the raw materials used in the method of the disclosure may be applied. Thus, the ratios of the amounts of the raw materials used in the method of the disclosure may be read as the ratios of the amounts of the components contained in the composition of the disclosure. In the method of the disclosure, the ratio of the total amount of the effective components used with respect to the total amount of the raw materials used, in terms of the weight ratio, may be read as the total content of the effective components in the composition of the disclosure. The content of each effective component in the composition of the disclosure may be within a range calculated based on the ratio between the total content of the effective components in the composition of the disclosure and the amount of the component contained in the composition of the disclosure.

The content of the bacterium in the composition of the disclosure may be, for example, 1×10⁴ cfu/g or more, 1×10⁵ cfu/g or more, 1×10⁶ cfu/g or more, or 1×10⁷ cfu/g or more, and may be, for example, 1×10¹³ cfu/g or less, 1×10¹² cfu/g or less, or 1×10¹¹ cfu/g or less. The content may be within a range defined by their combination. More specifically, the content of the bacterium in the composition of the disclosure may be, for example, 1×10⁴ to 1×10¹³ cfu/g, preferably 1×10⁶ to 1×10¹² cfu/g, more preferably 1×10⁷ to 1×10¹¹ cfu/g.

In the composition of the disclosure, the bacterium may be, for example, coated with the oil or fat and the emulsifier. More specifically, in the composition of the disclosure, the bacterium may be, for example, coated with a mixture of the oil or fat and the emulsifier. More specifically, in the composition of the disclosure, the bacterium may be, for example, coated with the oil or fat, and further coated with the emulsifier. More specifically, in the composition of the disclosure, the bacterium may be, for example, coated with the emulsifier, and further coated with the oil or fat.

The form of the bacterium composition of the disclosure is not limited. The composition of the disclosure may be in the form of, for example, a powder or paste.

The use of the composition of the disclosure is not limited. To the use of the composition of the disclosure, the description regarding the use of the bacterium composition obtained by the method of the disclosure may be applied. Thus, the composition of the disclosure may be used by, for example, addition to a food or beverage such as a powdered milk.

The method of producing the composition of the disclosure is not limited. The composition of the disclosure may be produced by, for example, the method of the disclosure described above. Thus, one mode of the composition of the disclosure may be a bacterium composition produced by the method of the disclosure.

### Examples

The disclosure is described below more concretely with reference to non-limiting Examples.

### [Example 1]

In the present Example, a heat resistance-improving effect on a bacterium by combined use of an oil or fat and an emulsifier was evaluated. As the bacterium, *Bifidobacterium longum* BB536 (NITE BP-02621; hereinafter also referred to as "BB536") was used. As the oil or fat, a fully hydrogenated palm kernel oil (K-40; manufactured by Taiyo Yushi Corp.) was used. As the emulsifier, a polyglycerin fatty acid ester (POEM RJ-38; manufactured by Riken Vitamin Co., Ltd.) was used. POEM RJ-38 is a polyglycerin fatty acid ester containing C18 (65%) and C16 (34%) constituent fatty acids.

BB536 was cultured, and the resulting bacterial cells were freeze-dried to obtain dried bacterial cells. After placing 50 g of the dried BB536 bacterial cells in BALANCE GRAN LBG-1L (rotation diameter of the stirring blade = 0.136 m; manufactured by Freund-Turbo Corporation), the raw materials described in Table 1 were added thereto with stirring at 2400 rpm. After the addition of the raw materials, stirring was carried out under the conditions described in Table 1, to obtain a paste-like product. When the stirring was carried out in two stages, the raw material for the first layer was added, and then stirring was carried out under the conditions for the first layer. Thereafter, the raw material for the second layer was added, and then stirring was carried out under the conditions for the second layer. The fully hydrogenated palm kernel oil was preliminarily melted by heating, and then added dropwise to the bacterial cells. The polyglycerin fatty acid ester was added as it is, as a powder, to the bacterial cells. The product was dispersed in hot water at 70°C at a concentration of 1.0% by weight, and the survival rate of BB536 was measured 1 minute later, to obtain an index of heat resistance of BB536. Unless otherwise specified, the same experimental conditions were used in the following Examples.

The results are shown in Table 1. When the fully hydrogenated palm kernel oil and the polyglycerin fatty acid ester were used in combination (Nos. 4 to 6), higher heat resistance was obtained compared to when no treatment was carried out (No. 1) and when the fully hydrogenated palm kernel oil or the polyglycerin fatty acid ester was used alone (Nos. 2 and 3). In particular, when the fully hydrogenated palm kernel oil and the polyglycerin fatty acid ester were mixed with the bacterium in two separate stages (Nos. 5 and 6), higher heat resistance was obtained compared to when the fully hydrogenated palm kernel oil and the polyglycerin fatty acid ester were added at the same time (No. 4). On the other hand, when the stirring temperature after the addition of the fully hydrogenated palm kernel oil was lower than the melting point of the fully hydrogenated palm kernel oil, the heat resistance was hardly improved (No. 7). Thus, it became clear that heat resistance of the bacterium can be improved by mixing the bacterium with the oil or fat and the emulsifier, and that the mixing of the bacterium with the oil or fat is preferably carried out at the melting point of the oil or fat or a higher temperature.

### [Table 1]

**Table 1: Evaluation results on heat resistance of Bifidobacterium longum BB536**

| **No.** | **Raw material** | **1st layer** | | | **2nd layer** | | | **Survival rate (%) after treatment at 70°C for 1 minute** |
|---|---|---|---|---|---|---|---|---|
| | | **Temperature (°C)** | **Rotation rate (rpm)** | **Time (minutes)** | **Temperature (°C)** | **Rotation rate (rpm)** | **Time (minutes)** | |
| **1** | **Untreated** | | | | | | | **<0.001** |
| **2** | **Fully hydrogenated palm kernel oil, 3 g** | **45** | **2400** | **3** | | | | **0.4** |
| **3** | **Polyglycerin fatty acid ester, 12.5 g** | **50** | **2400** | **2.5** | | | | **1.5** |
| **4** | **Fully hydrogenated palm kernel oil, 3 g Polyglycerin fatty acid ester, 12.5 g Simultaneous addition** | **45** | **2400** | **5** | | | | **4.1** |
| **5** | **1st layer: Fully hydrogenated palm kernel oil, 3 g 2nd layer: Polyglycerin fatty acid ester, 12.5 g** | **45** | **2400** | **3** | **45** | **2400** | **5** | **15.0** |
| **6** | **1st layer: Polyglycerin fatty acid ester, 3 g 2nd layer: Fully hydrogenated palm kernel oil, 3 g** | **50** | **2400** | **3** | **45** | **2400** | **5** | **13.8** |
| **7** | **1st layer: Polyglycerin fatty acid ester, 3 g 2nd layer: Fully hydrogenated palm kernel oil, 12.5 g** | **50** | **2400** | **3** | **30** | **2400** | **5** | **2.0** |

### [Example 2]

In the present Example, a heat resistance-improving effect on a bacterium by combined use of an oil or fat and an emulsifier was evaluated for when the emulsifier was added in various amounts. The same bacterium, oil or fat, and emulsifier as in Example 1 were used.

The raw materials and the stirring conditions described in Table 2 were used to perform stirring, to evaluate heat resistance of the bacterium.

The results are shown in Table 2. When the polyglycerin fatty acid ester was added in various amounts (Nos. 8 to 12), higher heat resistance was obtained as compared to when no treatment was carried out (No. 1). In particular, when the amount of the polyglycerin fatty acid ester added was 10 g to 15 g (Nos. 9 to 11), high heat resistance was obtained.

### [Table 2]

**Table 2: Evaluation results on heat resistance of Bifidobacterium longum BR536**

| **No.** | **Raw material** | **1st layer** | | | **2nd layer** | | | **Survival rate (%) after treatment at 70°C for 1 minute** |
|---|---|---|---|---|---|---|---|---|
| | | **Temperature (°C)** | **Rotation rate (rpm)** | **Time (minutes)** | **Temperature (°C)** | **Rotation rate (rpm)** | **Time (minutes)** | |
| **1** | **Untreated** | | | | | | | **<0.001** |
| **8** | **1st layer: Fully hydrogenated palm kernel oil, 3 g 2nd layer: Polyglycerin fatty acid ester, 7.5 g** | **45** | **2400** | **3** | **45** | **2400** | **5** | **4.7** |
| **9** | **1st layer: Fully hydrogenated palm kernel oil, 3 g 2nd layer: Polyglycerin fatty acid ester, 10 g** | **45** | **2400** | **3** | **45** | **2400** | **15** | **12.9** |
| **10** | **1st layer: Fully hydrogenated palm kernel oil, 3 g 2nd layer: Polyglycerin fatty acid ester, 12.5 g** | **45** | **2400** | **3** | **45** | **2400** | **10** | **15.9** |
| **11** | **1st layer: Fully hydrogenated palm kernel oil, 3 g 2nd layer: Polyglycerin fatty acid ester, 15 g** | **45** | **2400** | **3** | **45** | **2400** | **5** | **12.0** |
| **12** | **1st layer: Fully hydrogenated palm kernel oil, 3 g 2nd layer: Polyglycerin fatty acid ester, 17.5 g** | **45** | **2400** | **3** | **45** | **2400** | **5** | **2.6** |

### [Example 3]

In the present Example, a heat resistance-improving effect on a bacterium by combined use of an oil or fat and an emulsifier was evaluated for when another emulsifier was used. The same bacterium and oil or fat as in Example 1 were used. As the emulsifier, lecithin (Yelkin TS; manufactured by ADM Japan Ltd.) was used. Yelkin TS is a soybean lecithin in a liquid form.

The raw materials and the stirring conditions described in Table 3 were used to perform stirring, to evaluate heat resistance of the bacterium. The lecithin was added as it is dropwise to the bacterial cells.

The results are shown in Table 3. Also, when the lecithin was used as the emulsifier (Nos. 13 to 28), higher heat resistance was obtained compared to when no treatment was carried out (No. 1). Thus, it became clear that not only polyglycerin fatty acid esters, but also various emulsifiers are capable of producing the heat resistance-improving effect on the bacterium when they are used in combination with an oil or fat.

### [Table 3]

**Table 3: Evaluation results on heat resistance of Bifidobacterium longum BB536**

| **No.** | **Raw material** | **1st layer** | | | **2nd layer** | | | **Survival rate (%) after treatment at 70°C for 1 minute** |
|---|---|---|---|---|---|---|---|---|
| | | **Temperature (°C)** | **Rotatio n rate (rpm)** | **Time (minutes)** | **Temperature (°C)** | **Rotation rate (rpm)** | **Time (minutes)** | |
| **1** | **Untreated** | | | | | | | **<0.001** |
| **13** | **1st layer: Fully hydrogenated palm kernel oil, 3 g 2nd layer: Lecithin, 2.5 g** | **45** | **2400** | **3** | **45** | **2400** | **40** | **9.4** |
| **14** | **1st layer: Fully hydrogenated palm kernel oil, 3 g 2nd layer: Lecithin, 3.5 g** | **45** | **2400** | **3** | **45** | **2400** | **5** | **21.8** |
| **15** | **1st layer: Fully hydrogenated palm kernel oil, 3 g 2nd layer: Lecithin, 3.5 g** | **45** | **2400** | **3** | **45** | **2400** | **20** | **24.7** |
| **16** | **1st layer: Fully hydrogenated palm kernel oil, 3 g 2nd layer: Lecithin, 3.5 g** | **45** | **2400** | **3** | **45** | **2400** | **40** | **37.3** |
| **17** | **1st layer: Fully hydrogenated palm kernel oil, 3 g 2nd layer: Lecithin, 3.5 g** | **45** | **2400** | **3** | **45** | **2400** | **60** | **27.1** |
| **18** | **1st layer: Fully hydrogenated palm kernel oil, 3 g 2nd layer: Lecithin, 4.5 g** | **45** | **2400** | **3** | **45** | **2400** | **40** | **33.6** |
| **19** | **1st layer: Fully hydrogenated palm kernel oil, 3 g 2nd layer: Lecithin, 5.5 g** | **45** | **2400** | **3** | **45** | **2400** | **60** | **55.1** |
| **20** | **1st layer: Fully hydrogenated palm kernel oil, 3 g 2nd layer: Lecithin, 5.5 g** | **45** | **2400** | **3** | **45** | **2400** | **120** | **50.2** |
| **21** | **1st layer: Fully hydrogenated palm kernel oil, 3 g 2nd layer: Lecithin, 6.5 g** | **45** | **2400** | **3** | **45** | **2400** | **40** | **33.4** |
| **22** | **1st layer: Fully hydrogenated palm kernel oil, 3 g 2nd layer: Lecithin, 6.5 g** | **45** | **2400** | **3** | **45** | **2400** | **60** | **33.4** |
| **23** | **1st layer: Fully hydrogenated palm kernel oil, 3 g 2nd layer: Lecithin, 6.5 g** | **45** | **2400** | **3** | **45** | **2400** | **120** | **37.3** |
| **24** | **1st layer: Fully hydrogenated palm kernel oil, 3 g 2nd layer: Lecithin, 7.5 g** | **45** | **2400** | **3** | **45** | **2400** | **40** | **30.8** |
| **25** | **1st layer: Fully hydrogenated palm kernel oil, 3 g 2nd layer: Lecithin, 8.5 g** | **45** | **2400** | **3** | **45** | **2400** | **30** | **31.4** |
| **26** | **1st layer: Fully hydrogenated palm kernel oil, 3 g 2nd layer: Lecithin, 9.0 g** | **45** | **2400** | **3** | **45** | **2400** | **30** | **30.2** |
| **27** | **1st layer: Fully hydrogenated palm kernel oil, 3 g 2nd layer: Lecithin, 10.5 g** | **45** | **2400** | **3** | **45** | **2400** | **20** | **44.4** |
| **28** | **1st layer: Fully hydrogenated palm kernel oil, 3 g 2nd layer: Lecithin, 12.0 g** | **45** | **2400** | **3** | **45** | **2400** | **10** | **23.6** |

### [Example 4]

In the present Example, a heat resistance-improving effect on a bacterium by combined use of an oil or fat and an emulsifier was evaluated for when another bacterium was used. The same oil or fat and emulsifier as in Example 3 were used. As the bacterium, *Bifidobacterium breve* M-16V (NITE BP-02622; hereinafter also referred to as "M-16V") was used.

M-16V was cultured, and the bacterial cells were freeze-dried to obtain dried bacterial cells. With 50 g of the dried M-16V bacterial cells, the raw materials and the stirring conditions described in Table 4 were used to perform stirring, to evaluate heat resistance of the bacterium.

The results are shown in Table 4. Also, when *Bifidobacterium breve* was used as the bacterium (No. 30), higher heat resistance was obtained compared to when no treatment was carried out (No. 29). Thus, it became clear that, by combined use of the oil or fat and the emulsifier, the heat resistance-improving effect can be obtained not only for *Bifidobacterium longum,* but also for various bacteria.

### [Table 4]

**Table 4: Evaluation results on heat resistance of Bifidobacterium breve M-16V**

| **No.** | **Raw material** | **1st layer** | | | **2nd layer** | | | **Survival rate (%) after treatment at 70°C for 1 minute** |
|---|---|---|---|---|---|---|---|---|
| | | **Temperature (°C)** | **Rotation rate (rpm)** | **Time (minutes)** | **Temperature (°C)** | **Rotation rate (rpm)** | **Time (minutes)** | |
| **29** | **Untreated** | | | | | | | **<0.00001** |
| **30** | **1st layer: Fully hydrogenated palm kernel oil, 3 g 2nd layer: Lecithin, 8.8 g** | **45** | **2400** | **3** | **45** | **2400** | **60** | **29.0** |

### [Example 5]

In BALANCE GRAN LBG-1L (rotation diameter of the stirring blade = 0.136 m; manufactured by Freund-Turbo Corporation), 50 g of a bacterial powder obtained by freeze-drying of a culture of BB536 was placed, and 3 g of a fully hydrogenated rapeseed oil in a liquid form preliminarily melted by heating was added thereto with stirring at 2400 rpm. Stirring was then carried out at 45°C at a rotation speed of 2400 rpm for 3 minutes. Subsequently, 5 g of lecithin in a liquid form was added to the resulting mixture, and the mixture was further stirred at 45°C at a rotation speed of 2400 rpm for 3 minutes, to obtain a composition containing a bacterium having high heat resistance.

### [Example 6]

In BALANCE GRAN LBG-1L (rotation diameter of the stirring blade = 0.136 m; manufactured by Freund-Turbo Corporation), 50 g of a bacterial powder obtained by freeze-drying of a culture of BB536 was placed, and 3 g of a fully hydrogenated palm oil in a liquid form preliminarily melted by heating was added thereto with stirring at 2400 rpm. Stirring was then carried out at 45°C at a rotation speed of 2400 rpm for 3 minutes. Subsequently, 10 g of a monoglycerin fatty acid ester was added to the resulting mixture, and the mixture was further stirred at 45°C at a rotation speed of 2400 rpm for 5 minutes, to obtain a composition containing a bacterium having high heat resistance.

### [Example 7]

In BALANCE GRAN LBG-1L (rotation diameter of the stirring blade = 0.136 m; manufactured by Freund-Turbo Corporation), 50 g of a bacterial powder obtained by freeze-drying of a culture of M-16V was placed, and 3 g of a fully hydrogenated palm kernel oil in a liquid form preliminarily melted by heating was added thereto with stirring at 2400 rpm. Stirring was then carried out at 45°C at a rotation speed of 2400 rpm for 3 minutes. Subsequently, 10 g of a sucrose fatty acid ester was added to the resulting mixture, and the mixture was further stirred at 45°C at a rotation speed of 2400 rpm for 5 minutes, to obtain a composition containing a bacterium having high heat resistance.

### Industrial Applicability

By the disclosure, heat resistance of a bacterium can be improved.

## Claims

1. A method of producing a composition containing a bacterium, the method comprising the following Steps A and B:
(A) mixing the bacterium with an oil or fat; and
(B) mixing the bacterium with an emulsifier;
wherein the Step A is carried out at the melting point of the oil or fat or a higher temperature.

2. The method according to claim 1, wherein the Step A begins before or after beginning the Step B.

3. The method according to claim 1, wherein the Steps A and B begin at the same time.

4. The method according to any one of claims 1 to 3, wherein the oil or fat has a melting point of 50°C or lower.

5. The method according to any one of claims 1 to 4, wherein the oil or fat has a melting point of 35°C or higher.

6. The method according to any one of claims 1 to 5, wherein the oil or fat is a fully hydrogenated palm kernel oil.

7. The method according to any one of claims 1 to 6, wherein the Step A is carried out at a temperature of 50°C or lower.

8. The method according to any one of claims 1 to 7, wherein the Step A is carried out at a temperature of 40°C or higher.

9. The method according to any one of claims 1 to 8, wherein the emulsifier is a lecithin or polyglycerin fatty acid ester.

10. The method according to any one of claims 1 to 9, wherein the Step B is carried out at a temperature of 50°C or lower.

11. The method according to any one of claims 1 to 10, wherein the Step B is carried out at a temperature of 40°C or higher.

12. The method according to any one of claims 1 to 11, wherein the amount of the oil or fat is 1.5 to 4.5 g per 50 g dry cell weight of the bacterium.

13. The method according to any one of claims 1 to 12, wherein
the emulsifier is a polyglycerin fatty acid ester, and
the amount of the emulsifier is 8 to 17 g per 50 g dry cell weight of the bacterium.

14. The method according to any one of claims 1 to 12, wherein
the emulsifier is a lecithin, and
the amount of the emulsifier is 1 to 20 g per 50 g dry cell weight of the bacterium.

15. The method according to any one of claims 1 to 14, wherein the bacterium belongs to the genus *Bifidobacterium.*

16. The method according to any one of claims 1 to 15, wherein the bacterium is *Bifidobacterium longum* or *Bifidobacterium breve.*

17. The method according to any one of claims 1 to 16, wherein the bacterium is *Bifidobacterium longum* BB536 (NITE BP-02621) or *Bifidobacterium breve* M-16V (NITE BP-02622).

18. A method of improving heat resistance of a bacterium, the method comprising the following Steps A and B:
(A) mixing the bacterium with an oil or fat; and
(B) mixing the bacterium with an emulsifier;
wherein the Step A is carried out at the melting point of the oil or fat or a higher temperature.

19. A composition comprising a bacterium, an oil or fat, and an emulsifier.

20. The composition according to claim 19, wherein the bacterium is coated with the oil or fat and the emulsifier.

21. A powdered milk comprising:
a composition produced by the method according to any one of claims 1 to 17; or
the composition according to claim 19 or 20.

22. The powdered milk according to claim 21, which is a powdered infant formula milk.
